# EUROPÄISCHE PATENTSCHRIFT

(11) **EP 2 193 751 B1**
(45) Veröffentlichungstag und Bekanntmachung des Hinweises auf die Patenterteilung: **21.07.2010**
(21) Anmeldenummer: 08021054.5
(22) Anmeldetag: 04.12.2008
(51) Int. Cl.: A61B 17/132, A61F 13/00

(54) **Druckverband für die Arterie im Arm eines Patienten**
Pressure bandage for arteries in the arm of a patient
Bande compressive pour l'artère dans le bras d'un patient

(43) Veröffentlichungstag der Anmeldung: 09.06.2010
(73) Patentinhaber: Karl Werkmeister, Medizinische Leibbinden Inh. Hans-Jürgen Germerodt, 37281 Wanfried (DE)
(72) Erfinder: Östreicher, Ulrich, 37276 Meinhard (DE)
(74) Vertreter: Walther, Walther & Hinz GbR

(56) Entgegenhaltungen:
- WO-A-97/04821
- WO-A-03/099143
- DE-A1- 10 054 188
- US-A- 3 050 064
- US-A- 4 005 709
- US-A- 4 182 338
- US-A- 4 479 495
- US-A- 5 295 996
- US-A1- 2004 092 999

## Beschreibung

Die vorliegende Erfindung betrifft einen Druckverband für die Arterie im Arm eines Patienten, umfassend einen aus einem elastischen Material hergestellten Bandagenabschnitt, der an einem Ende ein Kissen aufweist, wobei das Kissen einen über dem Kissen angeordneten Druckkörper zeigt, wobei das andere Ende des Bandagenabschnittes ein Verschlussmittel aufweist.

Durch die Arterie fließt das Blut vom Herzen zurück in die Extremitäten des Körpers. Das heißt, die Arterie steht unter Überdruck. Dies im Gegensatz zu der Vene, die das Blut zu dem Herzen hin befördert. Zu unterschiedlichen Anlässen wird die Arterie punktiert. Das heißt, es wird mittels einer Nadel oder Kanüle ein Zugang in die Arterie gelegt. Wird der Zugang entfernt, mithin die Nadel aus der Arterie gezogen, so muss, um ein Verschließen der Arterie zu gewährleisten, Druck auf die Punktion in der Arterie ausgeübt werden. Hierzu sind sogenannte Druckverbände bekannt, die über ein Kissen speziell auf die Punktion der Arterie einen gewissen Druck ausüben. Das Kissen kann hierbei aus einem Stück eines Mullverbandes o. ä. gebildet sein.

Aus der US-A-4,005,709 ist ein Druckverband der eingangs genannten Art bekannt. Hiebei wird die Bandage über einen Druckkörper geführt und endseitig z. B. durch einen Klettbandverschluss verschlossen.

Gegenstand der Erfindung ist ein Druckverband der eingangs genannten Art, mit dem ein erhöhter Druck auf die Punktion einer Arterie ausübt werden kann, so dass innerhalb kürzerer Zeit sichergestellt ist, dass sich die Arterienwandung verschließt, ohne dass die generelle Blutzufuhr in den Unterarm des Patienten unterbunden wird. Das heißt es soll vermieden werden, dass Taubheitsgefühle, beispielsweise in der Hand, auftreten.

Die Aufgabe wird erfindungsgemäß dadurch gelöst, dass der Bandagenabschnitt im Bereich des Druckkörpers eine Öse zum Durchziehen des anderen Endes des Bandagenabschnittes mit dem Verschlussmittel aufweist, wobei die Öse derart an dem Bandagenabschnitt angeordnet ist, dass im geschlossenen Zustand des Verbandes der Bandagenabschnitt über den Druckkörper geführt ist. Hieraus wird Folgendes deutlich:

Durch den auf dem Kissen aufliegenden Druckkörper, beispielsweise in Form eines rollenförmigen Druckkörpers aus Hartschaumstoff, der auf einer Seite auch abgeflacht sein kann, um ihn in einer bestimmten Lage auf dem Kissen besser fixieren zu können, wird zum einen ein relativ hoher punktueller Druck auf die Punktion in der Arterie ausgeübt, wobei jedoch durch das Kissen unter dem Druckkörper zu beiden Seiten des Druckkörpers eine Verteilung der Kraft stattfindet. Hierdurch besteht nicht die Gefahr, dass die generelle Blutversorgung der Extremität, und hier insbesondere der Hand, so weit unterbunden wird, dass Taubheitsgefühle auftreten. Dadurch, dass der Bandagenabschnitt im Bereich des Druckkörpers eine Öse zum Durchziehen des anderen Endes des Bandagenabschnittes an dem Verschlussmittel aufweist, kann eine um den Arm geführte Schlaufe erzeugt werden, die die Möglichkeit eröffnet, den Druckverband stramm zu ziehen. Die Öse ist hierbei derart an dem Bandagenabschnitt angeordnet, dass im geschlossenen Zustand des Verbandes das andere Ende des Bandagenabschnittes über den Druckkörper geführt ist, um so auf den Druckkörper die gewünschte Kraft zum Verschluss der Punktion der Arterie auszuüben zu können. Um den Druckkörper auf dem Druckverband zu fixieren, ist eine Schlaufe für den Druckkörper vorgesehen. Hieraus wird deutlich, dass die Öse derart an dem Bandagenabschnitt angeordnet ist, dass im Verschlussfall der Bandagenabschnitt über die Schlaufe zur Aufnahme des Druckkörpers geführt ist.

Nach einem weiteren Merkmal ist vorgesehen, dass sich das Kissen an dem einen Ende in den Bandagenbereich erstreckt, d. h. dass das Kissen nicht nur im Bereich des Druckkörpers vorgesehen ist, sondern sich darüber hinaus über den Druckkörper hinaus erstreckt, was ebenfalls dem Ziel dient, bei stramm anliegendem Druckverband zu verhindern, dass die Blutzufuhr in die Hand unterbunden wird.

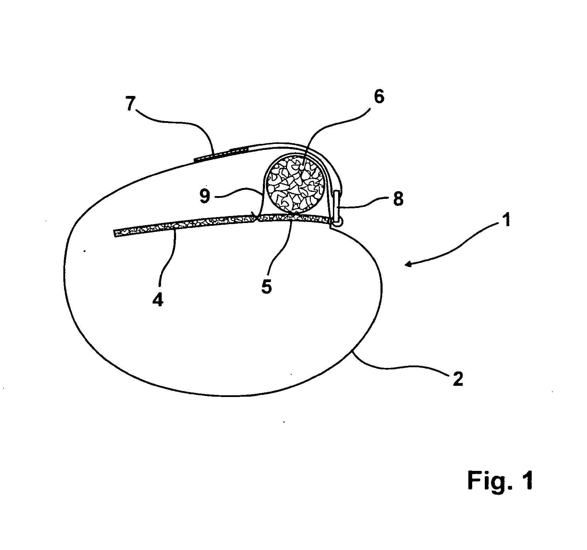

Anhand der Zeichnung wird die Erfindung nachstehend beispielhaft näher erläutert.

So zeigt der mit 1 bezeichnete Druckverband einen Bandagenabschnitt 2 aus einem elastisch nachgiebigen Material, das zudem noch atmungsaktiv ist, wobei an dem einen Ende des Bandagenabschnittes 2 ein in zwei Abschnitte 4 und 5 unterteiltes Kissen aus beispielsweise einem Abstandsgewirk vorgesehen ist, wobei der Kissenabschnitt 5 den Druckkörper 6 aus Hartschaumstoff, beispielsweise Styropor ®, aufweist. Der Bandagenabschnitt 2 erstreckt sich über diesen Druckkörper 6, d. h. der Bandagenabschnitt ist zur Bildung der Schlaufe 9 zu beiden Enden des Druckkörpers 6 mit dem Kissen verbunden, beispielsweise vernäht.

An dem anderen Ende des Bandagenabschnittes 2 ist ein Klettbandverschluss 7 vorgesehen, der derart ausgebildet ist, dass er auf dem Material des Bandagenabschnittes haftet.

Erkennbar ist ebenfalls die Öse 8 an dem einen Ende des Bandagenabschnittes 2, wobei die Öse 8 hinter dem Druckkörper 6 an dem Bandagenabschnitt 2 angeordnet ist, um beim Zuziehen des Druckverbandes einen möglichst hohen Druck über den Druckkörper dadurch auf die darunter befindliche Arterie ausüben zu können, dass der Bandagenabschnitt über dem Druckkörper verläuft. Der Druckkörper selbst ist rollenförmig ausgebildet, d. h. dass er im Bereich des Kissens 5 linienartig aufliegt, so dass die unter dem Kissen befindliche Arterie eine relativ hohe Flächenpressung erfährt, durch die Anordnung des Kissens unterhalb des Druckkörpers 6 gleichwohl eine gewisse Verteilung der Flächenkraft stattfindet, so dass auch bei nicht absolut genauer Positionierung des Druckkörpers über der Punktion der Arterie durch Anlegen des Druckverbandes sicher gewährleistet ist, dass durch den Druckkörper die Punktion der Arterie verschlossen wird. Das heißt, durch die Verwendung des rollenförmigen Hartschaumstoffes als Druckkörper in Verbindung mit einem sich darunter befindlichen Kissen werden zwei eigentlich konträre Vorgaben erfüllt, nämlich zum einen auf die Arterie nach Anlegen des Druckverbandes einen größtmöglichen Druck auszuüben, ohne dass die übrigen Blutgefäße übermäßig stark eingeschnürt werden und darüber hinaus weiterhin dafür zu sorgen, dass bei einer ungefähren Positionierung des Druckkörpers über der Arterie durch die Druckverteilung durch das Kissen sichergestellt ist, dass die Arterie mit der erforderlichen Druckkraft beaufschlagt wird.

Der Kissenabschnitt 4, der sich seitlich über den Bereich des Druckkörpers 6 erstreckt, dient dazu, eine zu starke Einschnürung des Gewebes bei Anlagen des Druckverbandes auch in diesem Bereich zu verhindern.

## Patentansprüche

1. Druckverband (1) für die Arterie im Arm eines Patienten, umfassend einen aus einem elastischen Material hergestellten Bandagenabschnitt (2), der an einem Ende ein Kissen (5) aufweist, wobei das Kissen (5) einen über dem Kissen angeordneten Druckkörper (6) zeigt, wobei das andere Ende des Bandagenabschnittes (2) ein Verschlussmittel (7) aufweist,
**dadurch gekennzeichnet,**
**dass** der Bandagenabschnitt (2) im Bereich des Druckkörpers (6) eine Öse (8) zum Durchziehen des anderen Endes des Bandagenabschnittes (2) mit dem Verschlussmittel (7) aufweist, wobei die Öse (8) derart an dem Bandagenabschnitt (2) angeordnet ist, dass im geschlossenen Zustand des Verbandes der Bandagenabschnitte (2) über den Druckkörper (6) geführt ist.

2. Druckverband nach Anspruch 1,
**dadurch gekennzeichnet,**
**dass** der Druckkörper (6) aus einem Hartschaumstoff hergestellt ist.

3. Druckverband nach einem der voranstehenden Ansprüche,
**dadurch gekennzeichnet,**
**dass** der Druckkörper (6) rollenförmig ausgebildet ist.

4. Druckverband nach einem der voranstehenden Ansprüche,
**dadurch gekennzeichnet,**
**dass** das elastische Material des Bandagenabschnitts (2) atmungsaktiv ist.

5. Druckverband nach einem der voranstehenden Ansprüche,
**dadurch gekennzeichnet,**
**dass** der Druckverband (1) eine Schlaufe (9) zur Aufnahme des Druckkörpers (6) aufweist.

6. Druckverband nach einem der voranstehenden Ansprüche,
**dadurch gekennzeichnet,**
**dass** das Kissen (4, 5) an dem einen Ende des Bandagenabschnittes (2) über den Druckkörper (6) verlängert ist, um ein Einschnüren des Gewebes des Armes weitgehend zu vermeiden.

## Claims

1. Pressure bandage (1) for the artery in the arm of a patient, comprising a bandage section (2), which is made of an elastic material and which has a cushion (5) at one end, wherein the cushion (5) has a pressure body (6) arranged above the cushion and wherein the other end of the bandage section (2) has a closure means (7), **characterised in that** the bandage section (2) has in the region of the pressure body (6) an eye (8) for drawing through the other end of the bandage section (2) with the closure means (7), wherein the eye (8) is so arranged at the bandage section (2) that in the closed state of the bandage the bandage section (2) is led over the pressure body (6).

2. Pressure bandage according to claim 1, **characterised in that** the pressure body (6) is made of a hard foam material.

3. Pressure bandage according to one of the preceding claims, **characterised in that** the pressure body (6) is of roll-shaped construction.

4. Pressure bandage according to any one of the preceding claims, **characterised in that** the elastic material of the bandage section (2) can breathe.

5. Pressure bandage according to any one of the preceding claims, **characterised in that** the pressure bandage (1) has a loop (9) for receiving the pressure body (6).

6. Pressure bandage according to any one of the preceding claims, **characterised in that** the cushion (4, 5) is prolonged at one end of the bandage section (2) beyond the pressure body (6) in order to substantially avoid cutting into the tissue of the arm.

## Revendications

1. Pansement compressif (1) pour les artères du bras d'un patient, comprenant un tronçon de bandage (2) fabriqué en une matière élastique et qui comporte un rembourrage (5) à une extrémité, dans lequel le rembourrage (5) porte un bloc de compression (6) disposé au-dessus du rembourrage, l'autre extrémité du tronçon de bandage (2) comportant un moyen de fermeture (7),
**caractérisé en ce que**
le tronçon de bandage (2) comporte dans la région du bloc de compression (6) un oeillet (8) dans lequel est enfilée l'autre extrémité du tronçon de bandage (2) qui porte le moyen de fermeture, l'oeillet (8) étant disposé sur le tronçon de bandage (2) de telle manière qu'à l'état fermé du pansement, le tronçon de bandage (2) passe au-dessus du bloc de compression (6).

2. Pansement compressif selon la revendication 1,
**caractérisé en ce que**
le bloc de compression (6) est fabriqué en une mousse dure.

3. Pansement compressif selon l'une des revendications précédentes,
**caractérisé en ce que**
le bloc de compression (6) est en forme de rouleau.

4. Pansement compressif selon l'une des revendications précédentes,
**caractérisé en ce que**
la matière élastique du tronçon de bandage (2) est du type respirant actif.

5. Pansement compressif selon l'une des revendications précédentes,
**caractérisé en ce que**
le pansement compressif (1) comporte une boucle (9) destinée à recevoir le bloc de compression (6).

6. Pansement compressif selon l'une des revendications précédentes,
**caractérisé en ce que,**
à une extrémité du tronçon de bandage (2), le rembourrage (4, 5) est prolongé au-delà du bloc de compression (6) pour éviter, dans une grande mesure, de serrer les tissus du bras.
